# EUROPEAN PATENT SPECIFICATION

(11) **EP 1 774 851 B1**
(45) Date of publication and mention of the grant of the patent: **13.07.2011**
(21) Application number: 06021345.1
(22) Date of filing: 11.10.2006
(51) Int. Cl.: A01M 19/00, A01M 17/00, A23L 3/3409, A61L 2/20

(54) **Disinfestation method particularly for works of art**
Verfahren zur Entwesung von Kunstwerken
Méthode pour désinfester des oeuvres d'art

(30) Priority: 14.10.2005 IT MI20051940
(43) Date of publication of application: 18.04.2007
(73) Proprietor: Finanziaria Unterland S.p.A., 39100 Bolzano (BZ) (IT)
(72) Inventor: Pruneri, Marco, 39040 - Ora (Bolzano) (IT)
(74) Representative: Forattini, Amelia

(56) References cited:
- EP-A- 1 388 284
- EP-A1- 0 963 694
- EP-A2- 0 320 012
- WO-A-2005/039654
- DE-A1- 19 545 926
- DE-A1- 19 646 991
- DE-A1- 19 706 842
- DE-A1- 19 706 844
- DE-C1- 19 627 084

## Description

The present invention relates to a disinfestation method particularly for works of art.

It is known to perform disinfestation of works of art from pests in a controlled atmosphere. These disinfestations are performed in sealed environments and are characterized by very low oxygen values of approximately 1%.

DE-A-197 06 844 discloses such a process, whereby a residual oxygen value below 4% by volume and a temperature of 30°C to 40°C are maintained.

This is done because past studies of the various entomology centers have focused on testing the effectiveness of treatments with low oxygen values on different families of insects.

Past studies have highlighted a close correlation between the duration of treatments with low residual oxygen percentages (1%, ..., 2%) and temperature values in the environments being treated.

Experiences and studies have confirmed that with temperatures below 18°C the metabolism of insects in their four stages of development (egg, larva, pupa and adult) slows down to the point of requiring treatment times which are so long as to be uneconomical.

The disadvantage of having to use such low residual oxygen values is that it is necessary to resort, for disinfestation treatments, to high-performance machines and to environments which have particularly extreme airtightness characteristics.

In practice, this forces to provide disinfestation environments with these characteristics and to move the objects to be disinfested from their usual location, place them within the disinfestation environment, and reposition them to the original site at the end of the disinfestation treatment.

This handling, in addition to being expensive in terms of time and personnel, exposes the objects to the risk of damage.

Another disadvantage of the current method is that it is not applicable to the treatment of large volumes and/or environments and is not applicable to objects that must be considered immovable, for example because they are too large and/or can be disassembled with a great expenditure of time and work.

The aim of the present invention is to provide a disinfestation method particularly for works of art which overcomes the drawbacks of the cited prior art.

An object of the invention is to provide a disinfestation method which can be used directly even within large environments to be treated, without having to provide specific environments with particularly critical seal characteristics.

Another object of the invention is to provide a method which is environmentally friendly and can be used in the vast majority of environments that are present in existing buildings.

An important object of the present invention is to provide a method that can be used for immovable objects as well.

Another object of the invention is to provide a method by means of which it is possible to use machines which have a lower performance and therefore are less bulky than the machines used with conventional methods.

Another object is to provide a method which is characterized by a low power consumption for the treatments.

Another object is to provide a method which allows to significantly reduce the total time of the intervention, which traditionally also includes the time for moving the objects and the time for providing a tight seal to the environment.

This aim and these and other objects, which will become better apparent hereinafter, are achieved by a disinfestation method particularly for works of art, comprising the steps of producing a moderate airtightness of an environment which contains the object to be treated, modifying the atmosphere of the environment so as to obtain residual oxygen values comprised between 8% and 13% by volume, and keeping the environment at temperatures comprised between 30°C and 40°C.

Further characteristics and advantages of the invention will become better apparent from the description of a preferred but not exclusive embodiment thereof.

The present Applicant has established a surprising increasing effectiveness of treatments with relatively high residual oxygen values (8%, ..., 13% by volume) and temperatures comprised between 30°C and 40°C for periods comprised between seven and fifteen days.

These interesting and surprising results have allowed to define a new method in environmentally-friendly disinfestation treatments.

The method, according to the present invention, consists in using residual oxygen values comprised indeed between 8% and 13% to perform disinfestations and in keeping the environments and/or objects to be disinfested at temperatures comprised between 30°C and 40°C for periods comprised between seven and fifteen days.

These conditions can be easily achieved in the vast majority of existing buildings.

In particular, in order to be able to maintain temperatures between 30°C and 40°C inside the environments to be treated, it is usually sufficient to operate and control the usual heating system of the room or building.

In particular in spring and autumn, and especially in the summer, keeping these temperature values for periods of even fifteen days entails no serious difficulty or high energy consumption.

Likewise, reaching and maintaining residual oxygen values comprised between 8% and 13% inside any closed environment does not entail particular technical difficulties or considerable energy consumption.

The advantages of the method according to the present invention are several.

The method according to the present invention can be directly applied within environments to be treated, even large ones, without having to provide custom environments with particularly critical airtightness characteristics.

This allows to apply the present environmentally-friendly method with low oxygen to the vast majority of the environments that are present in existing buildings.

The present method can also be used for immovable objects.

With the method according to the present invention it is possible to use machines which have a lower performance and are therefore less bulky.

Another important advantage of the present method is the reduced power consumption of the treatments.

The present method avoids moving the objects to be treated, consequently reducing disinfestation costs and the risks associated with handling fragile and precious objects.

Another advantage of the present invention is the significant reduction of total intervention times that traditionally included the time required for moving the objects and for establishing the hermetic environments used for the treatments.

In practice it has been found that the invention achieves the intended aim and objects.

This application claims the priority of Italian Patent Application No. M12005A001940, filed on October 14, 2005.

The method according to the invention is susceptible of numerous modifications and variations, within the scope of the appended claims.

The materials used, as well as the dimensions, may be any according to requirements and to the state of the art.

## Claims

1. A disinfestation method particularly for works of art, comprising the steps of producing a moderate airtightness of an environment which contains the object to be treated, modifying the atmosphere of said environment so as to obtain residual oxygen values comprised between 8% and 13% by volume, and keeping said environment at temperatures comprised between 30°C and 40°C.

2. The method according to claim 1, **characterized in that** the atmosphere of said, environment is maintained with oxygen values comprised between 8% and 13% by volume, at temperatures comprised between 30°C and 40°C, for a period of time comprised between 7 and 15 days.

3. The method according to claim 1 or 2, **characterized in that** the temperature in said environment is maintained by using the existing heating system in the rooms of the building.

4. The method according to one or more of the preceding claims, **characterized in that** said environment is established directly inside a room of a building and without particularly critical airtightness characteristics.

5. The method according to one or more of the preceding claims, **characterized in that** said environment is established around an immovable object to be disinfested.

## Patentansprüche

1. Verfahren zur Entwesung insbesondere von Kunstwerken, umfassend die Schritte des Herstellens einer mäßigen Luftdichtheit einer Umgebung, die das zu behandelnde Objekt enthält, des Modifizierens der Atmosphäre der Umgebung, so dass Restsauerstoffwerte zwischen 8 und 13 Vol.-% erhalten werden, und des Haltens der Umgebung auf Temperaturen zwischen 30°C und 40°C.

2. Verfahren nach Anspruch 1, **dadurch gekennzeichnet, dass** die Atmosphäre der Umgebung bei Sauerstoffwerten zwischen 8 und 13 Vol.-% für eine Dauer zwischen 7 und 15 Tagen bei Temperaturen zwischen 30°C und 40°C gehalten werden.

3. Verfahren nach Anspruch 1 oder 2, **dadurch gekennzeichnet, dass** die Temperatur in der Umgebung durch die Nutzung des vorhandenen Heizsystems in den Räumen des Gebäudes gehalten wird.

4. Verfahren nach einem oder mehreren der vorhergehenden Ansprüche, **dadurch gekennzeichnet, dass** die Umgebung direkt in einem Raum eines Gebäudes und ohne besonders kritische Luftdichtheitscharakteristiken geschaffen wird.

5. Verfahren nach einem oder mehreren der vorhergehenden Ansprüche, **dadurch gekennzeichnet, dass** die Umgebung rund um ein zu entwesendes unbewegliches Objekt geschaffen wird.

## Revendications

1. Procédé pour la désinfestation en particulier des oeuvres d'art, comprenant les étapes consistant à produire une étanchéité à l'air modérée d'un environnement qui contient l'objet devant être traité, à modifier l'atmosphère dudit environnement de façon à obtenir des valeurs d'oxygène résiduel comprises entre 8 % et 13 % en volume, et à maintenir ledit environnement à des températures comprises entre 30°C et 40°C.

2. Procédé selon la revendication 1, **caractérisé en ce que** l'atmosphère dudit environnement est maintenu à des valeurs d'oxygène comprises entre 8 % et 13 % en volume, à des températures comprises entre 30°C et 40°C, pendant une période de temps comprise entre 7 et 15 jours.

3. Procédé selon la revendication 1 ou 2, **caractérisé en ce que** la température dans ledit environnement est maintenue par utilisation du système de chauffage existant dans les pièces de l'immeuble.

4. Procédé selon une ou plusieurs des revendications précédentes, **caractérisé en ce que** ledit environnement est établi directement à l'intérieur d'une pièce d'un immeuble et sans caractéristiques d'étanchéité à l'air particulièrement critiques.

5. Procédé selon une ou plusieurs des revendications précédentes, **caractérisé en ce que** ledit environnement est établi autour d'un objet inamovible devant être désinfesté.
